(19) Europäisches Patentamt
European Patent Office
Office européen des brevets

(11) Publication number: 0 152 194
A2

(12) **EUROPEAN PATENT APPLICATION**

(21) Application number: 85300420.8

(22) Date of filing: 23.01.85

(51) Int. Cl.[4]: A 61 K 7/08

(30) Priority: 25.01.84 US 573780
17.08.84 US 642002

(43) Date of publication of application:
21.08.85 Bulletin 85/34

(84) Designated Contracting States:
AT BE CH DE FR GB IT LI NL

(71) Applicant: THE PROCTER & GAMBLE COMPANY
301 East Sixth Street
Cincinnati Ohio 45201(US)

(72) Inventor: Cobb, Daniel Scott
130 Albright Drive
Loveland Ohio 45140(US)

(74) Representative: Brooks, Maxim Courtney et al,
Procter & Gamble (NTC) Limited Whitley Road
Longbenton
Newcastle-upon-Tyne NE12 9TS(GB)

(54) **Shampoo compositions.**

(57) Shampoo compositions containing a quaternary ammonium compound, a betaine surfactant and a aminofunctional silicone and having a pH in the range of from 2 to about 3.75.

EP 0 152 194 A2

Croydon Printing Company Ltd

# SHAMPOO COMPOSITIONS

Daniel S. Cobb

## BACKGROUND ART

The desire to develop products which simultaneously clean and condition hair has long been present. While the desire has long been present, developing such products has presented innumerable problems. Generally the agents which condition hair best are cationic with one or more long fatty hydrocarbon chains. Hair being negatively charged will allow for the cationic portion to attach to the hair while the long fatty chain(s) provide for ease of combing and hair conditioning.

Cationic materials generally cannot be used with good cleaning anionic surfactants and still deliver good hair condition. This meant that other surfactants such as nonionics, amphoterics and zwitterionics were examined by workers in the field. Many of these efforts are reflected in patents issued in the conditioning shampoo area.

U.S. Patent 3,849,348, November 19, 1974 to Hewitt discloses conditioning shampoos containing betaine, cationic and amine oxide surfactants. U.S. Patent 3,697,452, October 10, 1972 to Olson et al. discloses shampoo compositions similar to those in Hewitt. Another patent to Hewitt is U.S. Patent 3,755,559, August 28, 1973 disclosing shampoos containing a tertiary amine oxide, a higher alkyl betaine and a soap. U.S. Patent 3,822,312, July 2, 1974 to Sato discloses shampoos containing a quaternary ammonium salt, a betaine and an additional additive. U.S. Patent 3,990,991, November 9, 1961 to Gerstein discloses shampoos containing amphoteric surfactants and quaternary ammonium compounds. U.S. Patent 4,080,310, March 21, 1978 to Ng et al. discloses shampoos containing an amphoteric surfactant, a cationic resin and having a pH as low as 3. U.S. Patent 4,132,679, January 2, 1979 to Tsutsumi et al. discloses shampoos containing a phosphoric acid ester salt and a betaine. U.S. Patent 4,231,903, November 4, 1980 to Lindemann et al. discloses shampoos containing a mixture of an amido betaine and a phosphobetaine. U.S. Patent 4,247,548, January 27, 1981 to Barker

discloses a conditioning shampoo containing a betaine, a polypropoxylated quaternary ammonium chloride surfactant and gum arabic. U.S. Patent 4,294,728, October 13, 1981 to Vanlerberghe et al. discloses shampoos containing a cationic, amphoteric or zwitterionic surfactant and a diol. U.S. Patent 4,329,335, May 11, 1981 to Su et al. discloses a shampoo composition containing a betaine, an amine oxide and a polymerized quaternary compound. U.S. Patent 4,181,634, January 1, 1980 to Kennedy et al. discloses shampoos containing a betaine and a bisquaternary compound.

While the above described references disclose compositions containing components of the type used in the present compositions, they do not teach or suggest totally satisfactory answers to the questions of good cleaning, conditioning and stability. It is believed that good cleaning with quaternary compounds is in part dependent on limiting the reacting of the quaternary with the fatty acids in sebum.

Additionally the use of quaternary compounds alone have been found deficient in some respects of hair conditioning. Specifically improvements in dry combing and body are desirable.

Applicants have surprisingly found that including an aminofunctional silicone in the present compositions can provide for improvements of the type described. References disclosing such materials but not in the present type of compositions include: European Patent Application 0078597, November 5, 1983 to Toray Silicone Company Ltd.; U.S. Patent 3,460,981, August 12, 1969 to Kell et al. disclosing an ice release composition containing an aminosilicone; U.S. Patent 4,185,087, January 22, 1980 to Morlino disclosing shampoo compositions containing a betaine surfactant and a quaternized organosilicon compound; U.S. Patent 4,342,742, August 3, 1982 to Sebag et al. disclosing aminofunctional silicones in hair care compositions; and U.S. Patent 4,416,794, November 22, 1983 to Barrat et al. disclosing dish rinsing compositions containing an amino-silane.

It is an object of the present invention to provide hair conditioning shampoo compositions which provide good cleaning and conditioning including improved dry combing and body. The

good cleaning relates to improved sebum emulsification as well as good lather.

It is a further object of the present invention to provide shampoo compositions containing particular betaine surfactants, quaternary ammonium compounds and aminofunctional silicones and having a pH in the range of from about 2 to about 3.75.

These and other objects will become more apparent from the detailed description which follows.

Unless otherwise indicated, all percentages and ratios herein are by weight.

## DISCLOSURE OF THE INVENTION

The compositions of the present invention comprise from about 5% to 70% of an amido betaine, from about 0.5% to about 10% of a quaternary ammonium or imidazolinium compound, from about 0.5% to about 10% of an aminofunctional silicone and from about 20% to about 94.0% water and having a pH in the range of from about 2 to about 3.75.

## DETAILED DESCRIPTON OF THE INVENTION

The essential as well as optional components of the present invention are described in detail below.

### Surfactant

The essential surfactants used in the compositions of the present invention are higher alkylamido betaines.

The betaines may be represented by the following structural formula:

$$R_1 - CONH(CH_2)_y - \overset{\displaystyle R_2 \atop |}{\underset{| \atop \displaystyle R_3}{N^+}} - R_4 - X^-$$

wherein $R_1$ is a long chain alkyl radical having from about 10 to about 18 carbon atoms, $R_2$ and $R_3$ are each alkyl radicals having from about 1 to about 3 carbon atoms, $R_4$ is an alkylene or hydroxy alkylene radical having from about 1 to about 4 carbon atoms, y is an integer from 1 to 4, and X is a carboxylate radical. $R_1$ may be a mixture of long chain alkyl radicals and may

contain one or more intermediate linkages or non-functional substituents such as hydroxyl or halogen radicals which do not affect the hydrophobic character of the radical. Examples of betaines useful herein include cocoamidopropyldimethylcarboxymethyl betaine, laurylamidopropyldimethylcarboxymethyl betaine among many others. In many instances the dimethylcarboxymethyl part of the designation is not included. Mixtures of the betaines are also acceptable.

The amount of surfactant is from about 5% to about 70%, preferably from about 10% to about 25%.

Quaternary Compound

The second essential component of the present invention is a quaternary ammonium, imidazolinium salt or mixtures of one or both.

Quaternary ammonium salts can have the formula:

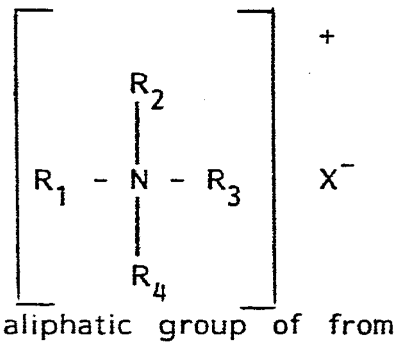

wherein $R_1$ is hydrogen, an aliphatic group of from 1 to 22 carbon atoms, or an aromatic, aryl or alkaryl group having 6 to 20 carbon atoms; $R_2$ is an aliphatic group having from 12 to 22 carbon atoms; $R_3$ and $R_4$ are each alkyl groups having from 1 to 3 carbon atoms; and X is an anion selected from halogen, acetate, phosphate, nitrate and methyl sulfate radicals.

Preferred quaternary ammonium salts are the dialkyldimethylammonium chlorides, wherein the alkyl groups have from 12 to 22 carbon atoms and are derived from long chain fatty acids, such as tallow or hydrogenated tallow. The term "tallow" refers to fatty alkyl groups derived from tallow fatty acids. Such fatty acids give rise to quaternary compounds wherein $R_1$ and $R_2$ have predominatel/ from 16 to 18 carbon atoms.

Representative examples of quaternary ammonium salts useful in this invention include ditallowdimethylammonium chloride, ditallowdimethylammonium methyl sulfate, dihexadecyldimethylam-

monium chloride, di(hydrogenated tallow)dimethylammonium chloride, dioctadecyldimethylammonium chloride, dieicosyldimethylammonium chloride, didocosyldimethylammonium chloride, di(hydrogenated tallow)dimethylammonium acetate, dihexadecyldiethylammonium chloride, dihexadecyldimethylammonium acetate, ditallowdipropylammonium phosphate, ditallowdimethylammonium nitrate, di(coconut-alkyl)dimethylammonium chloride; cetyltrimethylammonium chloride; stearyldimethylbenzylammonium chloride and myristylalkonium chloride.

Other quaternary ammonium salts useful herein are the compounds of the formula

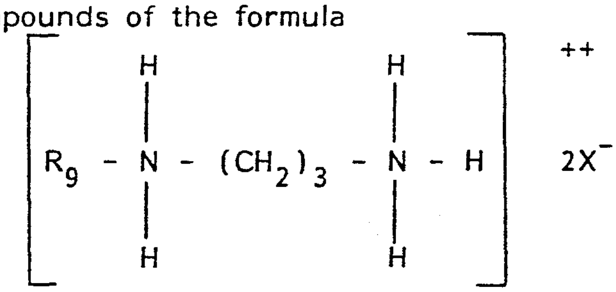

wherein $R_9$ is an aliphatic group having 16 to 22 carbon atoms and X is an anion as above defined. Tallow propanediamine hydrochloride is an example of this quaternary ammonium salt.

Quaternary imidazolinium salts have the formula

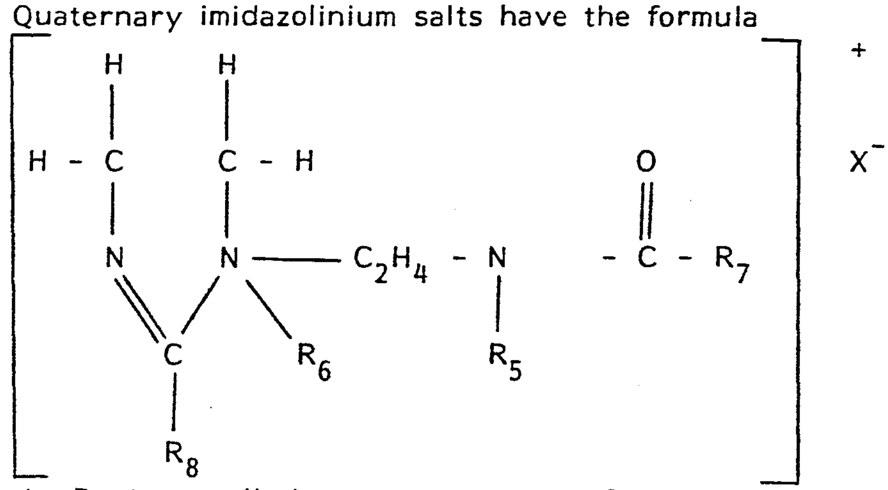

wherein $R_6$ is an alkyl group containing from 1 to 4, preferably from 1 to 2 carbon atoms; $R_5$ is an alkyl group containing from 1 to 4 carbon atoms or a hydrogen atom; $R_8$ is an alkyl group containing from 1 to 22, preferably at least 15 carbon atoms, or a hydrogen atom; $R_7$ is an alkyl group containing from 8 to 22,

preferably at least 15, carbon atoms; and X is an anion, preferably chloride. Other suitable anions include those disclosed with reference to the quaternary ammonium salts described hereinbefore.

Particularly preferred are those imidazolinium salts in which both $R_7$ and $R_8$ are alkyls of from 12 to 22 carbon atoms, e.g., 1-methyl-1-[(stearoylamide)ethyl]-2-heptadecyl-4,5-dihydroimidazolinium chloride; 1-methyl-1-[(palmitoylamide)ethyl]-2-octadecyl-4,5-dihydroimidazolinium chloride; and 1-methyl-1-[(tallowamide)-ethyl]-2-tallow-imidazolinium methyl sulfate.

Another imidazoline derivative favored for use in the present compositions is isostearyl ethylimidonium ethosulfate.

The quaternary salt is present at a level of from about 0.5% to about 10%, preferably from about 1% to about 6%.

Aminofunctional Silicone

The aminofunctional silicones useful in the present compositions correspond to the following structural formula:

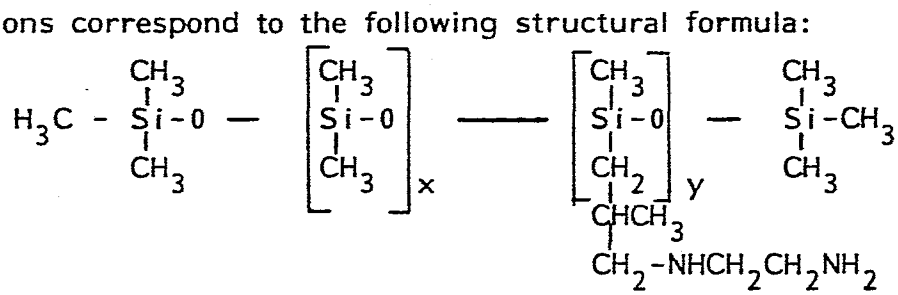

wherein the ratio of x:y may be from about 27.5:1 to about 160:1. The molecular weight may be from about 5,000 to about 30,000 as determined by gel permeation chromatography.

A particular material useful is Dow Corning Amodimethicone Q2-8075 which corresponds to the above formula and has a molecular weight of about 8000 with the ratio of x:y being about 49:1.

The amino functional material is used in the present compositions at a level of from about 0.25% to about 10%, preferably from about 1.0% to about 5.0%. Mixtures of the materials may also be used.

pH Adjustment

The compositions of the present invention have a pH below about 3.75 preferably from about 2.0 to about 3.75. The compositions are adjusted to this pH range with an acid buffer. The buffering capability is necessary since the pH of the diluted product (approximately 10:1 with water) when as applied to hair

should be near the range given. Suitable buffer solutions can be prepared, using for example agents such as citric acid, phosphoric acid, phthalic acid, glycine or mixtures thereof. In each case the proper buffering capacity is obtained by adjusting the final pH of the compositions to within the pH range indicated above. This may be done by using a strong acid or a strong base (e.g., HCl or NaOH) as may be needed. The most preferred agent is citric acid. The amount of buffer employed in the present compositions depends on the particular acid chosen but is generally from about 0.3% to about 6%, preferably from about 1% to about 5%.

Water

Water is the last essential component of the present invention and forms the remainder of the composition. It is generally present at a level of from about 20% to about 94%, preferably from about 65% to about 80%.

Optional Components

The shampoos herein can contain a variety of non-essential optional components suitable for rendering such compositions more stable and desirable. Such conventional optional ingredients are well known to those skilled in the art, e.g., preservatives such as benzyl alcohol, methyl paraben, propyl paraben and imidazolidinyl urea; betaine surfactants such as lauryl betaine in an amount up to about equal to the amount of the amidobetaine; thickeners and viscosity modifiers such as an ethoxylated amide of a long chain fatty acid (e.g., PEG-3-lauramide), sodium chloride, sodium sulfate, methylcellulose, polyvinyl alcohol, $C_{10}$ - $C_{14}$ alcohols (e.g., lauryl alcohol), and ethyl alcohol; suspending agents such as hydrogenated castor oil; opacifiers such as ethylene glycol distearate; perfumes; dyes; and, sequestering agents such as disodium ethylenediamine tetraacetate. Such agents, except for the betaine surfactants, generally are used individually at a level of from about 0.01% to about 10%.

## METHOD OF MANUFACTURE

The shampoos of the present invention may be made in a variety of ways. A preferred method is set forth in Example I.

## INDUSTRIAL APPLICABILITY

The present compositions are used in a conventional manner for cleaning hair. From about 0.1g to about 10g of the composition is applied to hair that has been wetted, generally with water, worked through the hair and then rinsed out.

The following Examples further describe and demonstrate the preferred embodiments within the scope of the present invention. The Examples are given solely for the purpose of illustration and are not to be construed as limitations of the present invention as many variations thereof are possible without departing from its spirit and scope.

## EXAMPLE I

The following composition representative of the present invention was prepared:

| COMPONENT | | WEIGHT % |
|---|---|---|
| Lexaine LM[1] | | 18.0 |
| Citric Acid | | 3.5 |
| Adogen 470DE[2] | | 2.0 |
| Amino Functional Silicone[5] | | 1.75 |
| Ethoxylated Amide[3] | | 4.5 |
| Variquat E-228[4] | | 2.0 |
| Lauryl Alcohol | | 0.5 |
| Ethylene Glycol Distearate | | 1.5 |
| Sodium Chloride | | 4.0 |
| Color, Perfume, Preservative and Water | q.s. | 100% |

[1] Lauramidopropyl betaine - Inolex Chemical Co.
[2] Di-(partially hydrogenated) tallow dimethylammonium chloride - Sherex Chemical Co.
[3] PEG-3 Alkyl (98% $C_{12}$) Amide (Mazamide C-2 Experimental) - Mazer Chemical Company
[4] Cetrimonium chloride - Sherex Chemical Company
[5] DC Q2 - 8075 offered by Dow Corning Corporation

Manufacturing Method for Example I

Lexaine LM and water are combined and heated to 160°F

under constant agitation. The ethoxylated amide, aminofunctional silicone, Ethylene Glycol Distearate, Adogen 470DE, Variquat E-228, lauryl alcohol, citric acid and sodium chloride are added in this sequence while the temperature is maintained. Once an essentially clear, homogeneous mixture is obtained, the composition is cooled to 120°F. At this temperature, the remaining ingredients and any make-up water are added to achieve the desired composition. The mixture is subsequently cooled to room temperature.

EXAMPLE II

The following composition representative of the present invention was also prepared.

| Component | | Weight % |
|---|---|---|
| Lexaine LM | | 18.00 |
| Ethoxylated amide as in Example I | | 4.50 |
| Dow Corning Q2-8075 Fluid | | 2.00 |
| Lauryl alcohol | | 1.00 |
| Adogen 470DE | | 2.00 |
| Variquat E-228 | | 2.00 |
| Citric acid | | 3.50 |
| Sodium chloride | | 4.00 |
| Color, perfume, preservative and water | q.s. | 100% |

EXAMPLE III

The following composition representative of the present invention was also prepared.

| Component | | Weight % |
|---|---|---|
| Lexaine LM | | 18.00 |
| Ethoxylated amide as in Example I | | 4.50 |
| Dow Corning Q2-8075 Fluid | | 1.75 |
| Lauryl alcohol | | 1.00 |
| Adogen 470DE | | 2.00 |
| Citric acid | | 3.50 |
| Sodium chloride | | 4.00 |
| Color, preservative, perfume and water | q.s. | 100% |

## EXAMPLE IV

Formulations similar to the composition of Example II were prepared and compared with that composition. The objective was to compare the compositions for dry combing ease. The compositions were as follows:

(a) Example II without the Dow Corning Fluid

(b) Example II without the Dow Corning Fluid but with 1% more of each of the quaternary compounds

(c) Example II with only 1% of the two quaternary compounds rather than 2% and Dow Corning Fluid

(d) Example II without the quaternary compounds

(e) Example II without the quaternary compounds or Dow Corning Fluid

Where the amounts of materials were different than that present in Example II, the differences were made up with water.

Several switches of hair (4g, 8 inches in length and clamped at top) were treated with Lilt Super Strength permanent waving lotion for 40 minutes in a straight configuration. After the neutralizer treatment the switches were rinsed and blow dried. Two switches were selected which were close in dry combing as judged by the test operator. The switches were then extracted with a 90% methylene chloride/10% methanol solvent mixture for one hour.

One switch was then treated (shampooed) with 0.4cc of an experimental product, rinsed, reapplied, rinsed and blow dried. The second switch was similarly treated with a second product.

After the switches were blow dried they were graded by 10 panelists for combing ease. The panelists were asked to select the easier of the two to comb.

Once the evaluations were complete, the switches were again subjected to the extraction procedure and the treatments were reversed.

The results of the comparisons, 20 panelist scores, were as follows:

| | | | |
|---|---|---|---|
| Example II versus (a) above | | 12:3 | 5np (no preference) |
| Example II versus (b) above | | 15:5 | |
| (d) above | versus (c) above | 9:10 | 1np |
| (b) above | versus (a) above | 13:3 | 4np |
| (d) above | versus (e) above | 10:8 | 2np |

It is seen that the composition representative of the present invention give unexpected improvements in dry combing ease. In the present systems the aminofunctional silicones perform better than additional quaternary material and better than what would be expected from the product with only the aminofunctional silicone.

CLAIMS

1. A shampoo composition comprising:
   (a) from about 0.5% to about 10% of a one or more cationic hair conditioning agents;
   (b) from about 5% to about 70% of one or more higher alkylamido betaines;
   (c) from about 0.25% to about 10% of one or more aminofunctional silicones having the following structural formula:

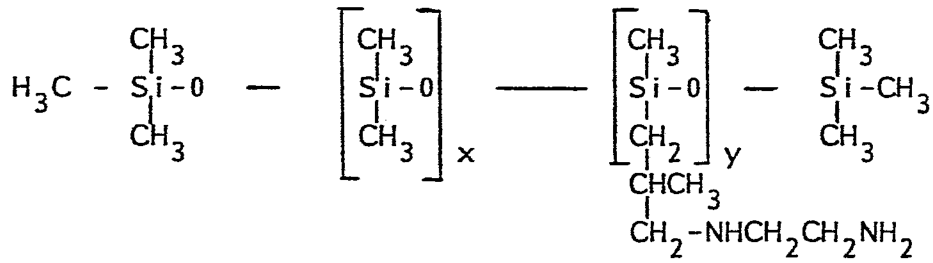

   wherein the ratio of x:y is from about 27.5:1 to about 160:1 and the molecular weight is from about 5,000 to about 30,000; and
   (d) water.
2. A shampoo composition according to Claim 1 wherein the cationic hair conditioning agent is selected from the group consisting of quaternary ammonium compounds, quaternary imidazolinium compounds and mixtures thereof.
3. A shampoo composition according to Claim 2 wherein the quaternary compound is present at a level of from about 1.0% to about 6.0%.
4. A shampoo composition according to Claim 2 or 3 wherein the higher alkylamido betaine is present at a level from about 10% to about 25%.
5. A shampoo composition according to any of Claims 2 to 4 wherein the aminofunctional silicone is present at a level of from about 1% to about 5%.
6. A shampoo composition according to any of Claims 2 to 5 wherein the quaternary ammonium compound is a dialkyldimethylammonium salt.

7. A shampoo composition according to any of Claims 2 to 6 wherein the higher alkylamido betaine is a higher alkylamidopropyl betaine.

8. A shampoo composition according to any of Claims 2 to 7 wherein the aminofunctional silicone has a molecular weight of about 8000 and the ratio of x:y is about 49:1.

9. A shampoo composition according to any of Claims 2 to 8 wherein the quaternary ammonium compound is ditallowdimethylammonium chloride.

10. A shampoo composition according to any of Claims 2 to 9 wherein the higher alkylamido betaine is cocamidopropyl betaine.